# EUROPEAN PATENT APPLICATION

(11) **EP 4 790 069 A1**
(43) Date of publication of application: **12.08.2026**
(21) Application number: 24874650.5
(22) Date of filing: 02.10.2024
(51) Int. Cl.: C07K 16/24, C07K 19/00, C12N 5/10, C12N 15/13

(54) **HUMAN CD153-TARGETING ANTIBODY AND CHIMERIC ANTIGEN RECEPTOR**

(30) Priority: 03.10.2023 US 202363542229 P; 14.06.2024 US 202463660004 P
(71) Applicant: Kyoto University, Kyoto-shi, Kyoto 606-8501 (JP); Cyn-K Bio, Inc., Kyoto-shi, Kyoto 606-8303 (JP)
(72) Inventor: HATTORI, Masakazu, Kyoto-shi, Kyoto 606-8501 (JP); FUKUSHIMA, Yuji, Kyoto-shi, Kyoto 606-8501 (JP); UENO, Ryuji, Easton, Maryland 21601 (US)
(74) Representative: Kraus & Lederer PartGmbB
(86) International application number: PCT/JP2024/035314
(87) International publication number: WO 2025/075049

(57) **Abstract**

The present application provides an anti-human CD153 monoclonal antibody, or a binding fragment thereof, which specifically binds to human CD153 and inhibits the intermolecular interaction between human CD153 and CD30. The present application further provides a chimeric antigen receptor comprising an extracellular antigen-binding domain, a transmembrane domain, and an intracellular signaling domain, wherein the extracellular antigen-binding domain comprises a single-chain antibody having the same heavy chain hypervariable regions and light chain hypervariable regions as those of the monoclonal antibody of the present application, and a CAR-T cell expressing the chimeric antigen receptor. The monoclonal antibody, the binding fragment thereof, and the CAR-T cell of the present application are useful for the treatment or prevention of diseases associated with CD153, such as multiple sclerosis.

## Description

### [Technical Field]

The present disclosure relates to an antibody and a chimeric antigen receptor targeting human CD153.

### [Background Art]

T cell population referred to as senescence-associated T cells (SA-T cells) are increased with age. SA-T cells have been known to cause the development and exacerbation of inflammatory diseases. Unlike normal T cells, SA-T cells are unable to proliferate or become activated in response to T cell antigen receptor (TCR) stimulation, while exhibit characteristics specific to senescent cells, i.e., secret large amounts of inflammatory factors. A vaccine comprising a partial amino acid sequence of CD153 as an immunogen has been proposed as a vaccine for the prevention or treatment of abnormal glucose metabolism targeting SA-T cells (Patent Literature 1).

Multiple sclerosis (MS) is a chronic inflammatory demyelinating disease of the central nervous system, characterized by the occurrence of multiple lesions disseminated in time and space. Diagnosis is generally made by demonstrating temporal and spatial dissemination of lesions through detailed medical history taking and neurological examinations over time, and by excluding other diseases. Although the cause of MS remains unclear, infiltration of lymphocytes and macrophages into lesions is observed, and demyelination is considered to occur due to inflammation mediated through autoimmune mechanisms. In addition, involvement of genetic and environmental factors has been suggested based on racial differences, but this has not been clearly elucidated.

Treatment of MS consists of treatment during acute exacerbation, treatment for prevention of relapse and progression, symptomatic treatment during acute and chronic phases, and rehabilitation. However, there is still no reliable method that can prevent relapse or disease progression of MS. MS affects young adults and follows a long-term course with repeated relapses and remissions. In many cases, relatively severe impairment remains in the optic nerve, spinal cord, and cerebellum, and there are not a few cases in which activities of daily living (ADL: Active Daily Living) are markedly reduced. The development of highly effective antibodies and vaccines useful for elucidating the mechanisms of MS and for treating MS is strongly desired.

Patent Literature 2 proposes the use of an antibody specific for CD30L, namely CD153, for the treatment of multiple sclerosis; however, the effect thereof is limited.

CAR-T cells are T cells in which a CAR composed of a "single-chain antibody (scFv) sequence" and an "activation signaling domain" is forcibly expressed in peripheral T cells, and they have a high ability of killing target cells. It has been suggested that intravenously administered CAR-T cells can cross the blood-brain barrier (BBB) and reach the central nervous system, and that local administration of CAR-T cells is also possible; therefore, CAR-T cells are suggested to be effective for elimination of target cells in the central nervous system (Non-Patent Document 1).

### [Citation List]

### [Patent Literatures]

[PLT 1] WO2020/096046
[PLT 2] U.S. Patent No. 6,652,854

### [Non-Patent Literature]

[NPL 1] Huang et al., 2023, Mol Cancer 22:22

The patent literatures and non-patent literature are herein incorporated by reference and constitute a part of this specification.

### Summary of the Invention

### Problems to be Solved by the Invention

An object of the present disclosure is to provide a method for the treatment of multiple sclerosis.

Another object of the present disclosure is to provide an anti-human CD153 monoclonal antibody or a binding fragment thereof that specifically binds to human CD153 and inhibits the interaction between CD153 and CD30.

Another object of the present disclosure is to provide a chimeric antigen receptor (CAR) that specifically binds to human CD153 and is capable of eliminating or killing cells expressing human CD153, and to provide a CAR-T cell expressing such CAR.

### Means for Solving the Problems

In the present disclosure, there is provided an anti-human CD153 monoclonal antibody, or a binding fragment thereof, that specifically binds to human CD153 and inhibits the interaction between CD153 and CD30. Examples of the anti-human CD153 monoclonal antibody include antibodies having the heavy chain complementarity determining regions (CDR-H1, CDR-H2, CDR-H3) and light chain complementarity determining regions (CDR-L1, CDR-L2, CDR-L3) shown in Table 1 below.

**[Table 1]**

| | | SEQ ID NO | | | SEQ ID NO |
|---|---|---|---|---|---|
| CDR-H1 | NYWLG | 1 | CDR-L1 | SASSSVNYMH | 4 |
| CDR-H2 | DIYPGNYNSYSNEKFKG | 2 | CDR-L2 | STSDLAS | 5 |
| CDR-H3 | IYGNYWIFDV | 3 | CDR-L3 | QQRSSYPLT | 6 |

The anti-human CD153 monoclonal antibody of the present disclosure is useful for treating or preventing diseases associated with CD153 in a subject.

The present disclosure also provides a chimeric antigen receptor (CAR) capable of specifically binding to CD153. The chimeric antigen receptor of the present disclosure comprises an extracellular antigen-binding domain, a transmembrane domain, and an intracellular signaling domain, and the extracellular antigen-binding domain comprises a single-chain antibody comprising the heavy chain complementarity determining regions and light chain complementarity determining regions of human CD153 shown in Table 1.

The present disclosure further provides a CAR-T cell expressing the CAR and having antigen-specific killer activity. The CAR-T cell of the present disclosure is useful for treating and preventing a disease associated with CD153 in a subject.

The present disclosure also provides a method for treating or preventing a disease associated with CD153 in a subject, comprising administering an effective amount of the anti-human CD153 monoclonal antibody or binding fragment thereof provided by the present disclosure to the subject in need of the treatment.

The present disclosure also provides a method for treating or preventing a disease associated with CD153 in a subject, comprising administering the CAR-T cell of the present disclosure to the subject in need of the treatment.

The present disclosure also provides use of the anti-human CD153 monoclonal antibody or binding fragment thereof provided by the present disclosure for manufacture of a medicament for treating or preventing a disease associated with CD153 in a subject.

The present disclosure also provides use of the CAR-T cell of the present disclosure for manufacture of a medicament for treating or preventing a disease associated with CD153 in a subject.

### Brief Description of the Drawings

[Fig. 1]
   Schematic diagram of the multiple sclerosis model in the Reference Example.
[Fig. 2]
   Graph showing the results of the Reference Example.
[Fig. 3]
   Reactivity of each anti-human monoclonal antibody obtained in Example 1 with human CD153.
   The average values of two wells are shown.
[Fig. 4]
   Reactivity of each anti-human monoclonal antibody obtained in Example 1 with human CD153.
   Results measured at an antibody concentration of 10 µg/mL are shown.
[Fig. 5]
   Reactivity of each anti-human monoclonal antibody obtained in Example 1 with mouse CD153.
   The average values of two wells are shown.
[Fig. 6]
   Reactivity of each anti-human monoclonal antibody obtained in Example 1 with mouse CD153.
   The average values of two wells are shown.
[Fig. 7]
   Reactivity of each anti-human monoclonal antibody obtained in Example 1 with mouse CD153.
   Results measured at an antibody concentration of 10 µg/mL are shown.
[Fig. 8]
   Inhibitory effects of each anti-human monoclonal antibody obtained in Example 1 on the intermolecular interaction between human CD153 and CD30.
[Fig. 9]
   Inhibitory effects of each anti-human monoclonal antibody obtained in Example 1 on the intermolecular interaction between human CD153 and CD30.
   Results measured at an antibody concentration of 10 µg/mL are shown.
[Fig. 10]
   Reactivity of each anti-human monoclonal antibody obtained in Example 1 with canine and feline CD153 (Example 3).
[Fig. 11]
   Killing effect of mCD153-CAR-T cells specific for mouse CD153 obtained in Example 4 on cells expressing mouse CD153 (Example 5).

### Mode for Carrying Out the Invention

### Definitions

In the present specification and claims, "effective amount" means the amount of an agent required to cure a disease or to induce remission of symptoms thereof in a subject compared to the subject when untreated. The amount of active ingredient used in practicing the present disclosure for prevention or treatment of a disease varies depending on the mode of administration, age, body weight, and overall health of the subject. Ultimately, the attending physician or veterinarian will determine the appropriate amount and dosage regimen. Such amount is referred to as an "effective" amount.

In the present specification and claims, "subject" means mammals including, but not limited to, humans and non-human mammals such as cattle, horses, dogs, sheep, and cats.

In the present specification and claims, the terms "treat", "treating", and "treatment" refer to alleviating or inducing remission of a disease and/or symptoms associated therewith.

In the present specification and claims, the terms "prevent", "preventing", "prevention", and "prophylactic treatment" include reducing the likelihood of onset in a subject who does not yet exhibit symptoms but is at risk of developing the disease, and also include suppressing progression of symptoms that have already developed in a subject.

As used herein, "pharmaceutically acceptable carrier" means one or more compatible solid or liquid fillers, diluents, or encapsulating substances suitable for administration to humans or other vertebrates, including aqueous solvents (e.g., water, alcoholic/aqueous solutions, saline, parenteral media such as sodium chloride and Ringer's dextrose), nonaqueous solvents (e.g., propylene glycol, polyethylene glycol, vegetable oils, injectable organic esters such as ethyl oleate), dispersing media, coating agents, surfactants, antioxidants, preservatives (e.g., antibacterial or antifungal agents, antioxidants, chelating agents, inert gases), isotonic agents, absorption delaying agents, salts, drugs, stabilizers, gels, binders, excipients, disintegrants, lubricants, sweeteners, flavoring agents, dyes, infusion fluids and nutritional supplements, and combinations thereof. The pH and exact concentration of various components in pharmaceutical compositions are adjusted according to well-known parameters.

The present disclosure provides an anti-human CD153 monoclonal antibody, or a binding fragment thereof, that specifically binds to human CD153 and inhibits the interaction between CD153 and CD30. The present disclosure also provides a pharmaceutical composition comprising the monoclonal antibody or binding fragment thereof.

The monoclonal antibody or binding fragment thereof provided in the present disclosure is useful for treatment of a disease associated with CD153 in a subject. Diseases in which CD153 is considered to be involved include, for example, central nervous system inflammatory demyelinating diseases including multiple sclerosis, neuromyelitis optica spectrum disorder (NMOSD), MOG antibody-associated disease (MOGAD), and Balo's concentric sclerosis (BCS); peripheral nervous system inflammatory demyelinating diseases including chronic inflammatory demyelinating polyneuropathy (CIDP); autoimmune neuropathies including Fisher syndrome (MFS); autoimmune rheumatic diseases including rheumatoid arthritis, systemic lupus erythematosus, Sjögren's syndrome, systemic sclerosis, and ankylosing spondylitis; inflammatory bowel diseases including ulcerative colitis and Crohn's disease; metabolic diseases including diabetes such as type 1 diabetes and type 2 diabetes; and chronic nephritis and renal failure, for example age-related chronic renal failure.

It has been confirmed that the monoclonal antibody having the combination of CDRs shown in Table 1 provided in the present disclosure binds not only to human CD153 but also to feline and canine CD153. Accordingly, the anti-human CD153 monoclonal antibody of the present disclosure, or a binding fragment thereof, can also be used for treatment of diseases associated with CD153 in a dog or cat subject.

The monoclonal antibody or binding fragment thereof provided in the present disclosure is particularly useful for treatment of multiple sclerosis. Clinical types of multiple sclerosis to be treated include, but are not limited to, relapsing-remitting type, secondary progressive type, primary progressive type, and clinically isolated syndrome.

As used herein, the term "antibody" refers to an immunoglobulin polypeptide molecule that binds to an antigen. A naturally occurring full-length antibody is an immunoglobulin molecule comprising two heavy (H) chains and two light (L) chains interconnected by disulfide bonds. The amino-terminal portion of each chain includes a variable region of about 100 to about 110 amino acids primarily responsible for antigen recognition through complementarity determining regions (CDRs) contained therein. The carboxy-terminal portion of each chain defines a constant region primarily responsible for effector function.

Between the CDRs, more conserved regions referred to as framework regions (FRs) are interspersed. Each light chain variable region (LCVR, also known as VL) and heavy chain variable region (HCVR, also known as VH) is composed of three CDRs and four FRs, arranged in the following order from amino terminus to carboxy terminus: FR1, CDR1, FR2, CDR2, FR3, CDR3, FR4. The three CDRs of the light chain are referred to as "CDR-L1, CDR-L2, and CDR-L3", and the three CDRs of the heavy chain are referred to as "CDR-H1, CDR-H2, and CDR-H3." The CDRs include the majority of residues that form specific interactions with the antigen.

As used herein, the term "monoclonal antibody" (mAb) refers to an antibody derived from a single copy or clone, including, for example, any eukaryotic organism, prokaryotic organism, or phage clone. The mAb of the present disclosure preferably exists in a uniform or substantially uniform population. A complete mAb includes two heavy chains and two light chains. Monoclonal antibodies may be produced by, for example, hybridoma technology, recombinant technology, phage display technology, synthetic technology such as CDR grafting, or combinations of such technologies or other technologies known in the art.

As used herein, the term "humanized antibody" refers to a monoclonal antibody having the combination of heavy chain and light chain CDRs provided in the present disclosure, wherein the framework regions surrounding the CDRs are generated and/or manipulated so as to be substantially similar or identical to human variant framework sequences. Humanization of antibodies is well known in the art. The humanized antibodies provided herein may be intentionally modified compared to the natural sequence, for example in the constant region, in order to alter effector properties or biological functional properties, or biophysical properties, particularly stability, developability, and/or solubility.

In the present disclosure, the "binding fragment" of an antibody includes, for example, Fab fragments, Fab' fragments, F(ab')2 fragments, and single-chain Fv fragments, which bind to the same antigen as the antibody and exhibit functional activity similar to that of the antibody.

The term "framework region" or "framework sequence" refers to any one of framework regions 1 to 4. Manipulated humanized antibodies and antigen-binding fragments thereof included in the present disclosure include molecules in which any one or more of framework regions 1 to 4 is substantially or completely of human (that is, any possible combination of individual framework regions substantially or completely human framework regions 1 to 4 may be present). For example, this includes molecules in which framework regions 1 and 2, framework regions 1 and 3, framework regions 1, 2, and 3, are substantially or completely of human. A substantially human framework has at least about 80% sequence identity to a known human germline framework sequence. Human germline framework sequences may be obtained from ImMunoGeneTics (IMGT) or from "The 20 Immunoglobulin FactsBook" by Marie-Paule Lefranc and Gerard Lefranc (Academic Press, 2001, ISBN 012441351).

As used herein, the terms "caninized antibody" and "felinized antibody" refer to monoclonal antibodies having the combination of heavy chain and light chain CDRs provided in the present disclosure, wherein the framework regions surrounding the CDRs are generated and/or manipulated so as to be substantially similar or identical to canine or feline variant framework sequences. In the case of caninized or felinized antibodies, the descriptions of binding fragments, framework regions, and framework sequences correspond to those for humanized antibodies with "human" read as "dog" or "cat."

The sequences of CDRs may be defined by various methods including Kabat, Chothia, AbM, contact, and IMGT definitions. Unless otherwise explicitly indicated, the CDRs in the present disclosure are identified according to the Kabat definition.

The present disclosure also provides a polynucleotide encoding the monoclonal antibody or antigen-binding fragment thereof of the present disclosure, and an expression vector comprising such polynucleotide.

In another aspect, the present disclosure provides a chimeric antigen receptor capable of specifically binding to human CD153.

A "chimeric antigen receptor (CAR)" is a polypeptide including an extracellular antigen-binding domain, a transmembrane domain, and an intracellular signaling domain. The intracellular signaling domain includes a functional signaling domain derived from a stimulatory molecule and/or a co-stimulatory molecule. As the extracellular antigen-binding domain of the CAR of the present disclosure, an scFv sequence having the heavy chain variable region and light chain variable region including the respective CDRs of the anti-human CD153 antibody shown in Table 1 provided herein is preferably used.

An "scFv" is a fusion protein including at least one antibody fragment including a light chain variable region and at least one antibody fragment including a heavy chain variable region, wherein the light chain and heavy chain variable regions are linked in close proximity by a linker and can be expressed as a single-chain polypeptide. The scFv retains the specificity of the antibody from which it is derived. The scFv may be configured such that the light chain variable region and heavy chain variable region are linked via a linker in either order at the N-terminal and C-terminal ends of the polypeptide.

The extracellular antigen-binding domain may be linked to the transmembrane domain by a hinge sequence. The intracellular signaling domain may include, for example, a functional signaling domain that may be the intracellular domain of CD3ζ. Furthermore, if desired, one or more co-stimulatory intracellular domains that provide co-stimulation of T cells upon binding of the antigen-binding domain to antigen may be further included. Examples of co-stimulatory intracellular domains include CD137, CD27, ICOS, and CD28.

The present disclosure provides a CAR and a polynucleotide encoding the CAR. Furthermore, the present disclosure provides a vector comprising the polynucleotide encoding the CAR.

The present disclosure further provides a CAR-T cell expressing the CAR of the present disclosure and having antigen-specific killer activity. A CAR-T cell can be obtained by expressing the CAR using a vector comprising a polynucleotide encoding the CAR in T cells isolated from peripheral blood or in T cells differentiated from pluripotent stem cells.

From experiments using gene-deficient mice, the present inventors reported that a unique functional interaction between CD153 molecules expressed on SA-T cells and CD30 molecules expressed on a very small number of autoreactive B cells is involved in the age-associated increase of SA-T cells. Through the interaction between CD153 and CD30, SA-T cells recover proliferative and activation responses via TCR, and autoreactive B cells proliferate and differentiate to produce autoantibodies. In other words, through bidirectional signaling between CD153 and CD30, SA-T cells and autoreactive B cells mutually stimulate and activate each other and proliferate. When an antibody (17D) that specifically binds to CD153 and blocks the interaction between CD153 and CD30 was administered to mice, it was confirmed that the age-associated increase of SA-T cells and the accompanying increase of autoantibodies and inflammatory factors were both significantly suppressed.

In addition, it is known that in model mice that develop systemic lupus erythematosus (SLE) and die due to renal failure, a marked increase of SA-T cells is observed from an early stage. When the 17D antibody was administered to such model mice, strong preventive effects on the onset of SLE pathology and therapeutic effects after onset were observed, and a clear improvement in survival rate was confirmed (Fukushima et al., 2022, Cell Reports 40, 111373; the contents of this document constitute part of the present application by reference).

The present inventors confirmed the effect of the mouse anti-mouse CD153 monoclonal antibody 17D on a multiple sclerosis model animal using the antibody. The relationship between increase of SA-T cells and multiple sclerosis has not been reported.

In the present disclosure, examples of anti-human CD153 antibodies that specifically bind to human CD153 and inhibit the intermolecular interaction between CD153 and CD30 include antibodies having the CDRs shown in Table 1 above. The antibodies of the present disclosure may be humanized, caninized, or felinized antibodies produced by conventional methods. Antibodies that compete for binding to human CD153 with the anti-human CD153 monoclonal antibody having the above CDRs, and humanized, caninized, or felinized antibodies thereof, are also exemplified.

As "anti-human CD153 antibodies that specifically bind to human CD153 and inhibit the intermolecular interaction between human CD153 and CD30" in the present disclosure, other antibodies having binding ability to the epitope recognized by the anti-human CD153 monoclonal antibody having the above CDRs, and humanized, caninized, or felinized antibodies thereof, are also exemplified.

The antibody or binding fragment of the present disclosure may be provided as a multi-specific antibody or fragment thereof that includes, in addition to a portion that specifically binds to human CD153, a portion that binds to a different antigen.

The antibody or antibody fragment of the present disclosure may have a drug conjugated thereto. The drug may be a low molecular weight compound, peptide, polynucleotide, or the like.

The antibody or binding fragment provided in this embodiment may be provided as a pharmaceutical composition, for example, a pharmaceutical composition including the antibody and a pharmaceutically acceptable carrier. The pharmaceutical composition is administered in an effective amount to a subject having a disease associated with CD153, for example multiple sclerosis, or to a subject suspected of having such disease. The pharmaceutical composition may be administered to a subject by intravenous (IV) administration (e.g., by injection or infusion), intramuscular administration, subcutaneous (SC) administration, parenteral administration, intrathecal administration, or epidermal administration.

The CAR-T cell provided in the present disclosure may also be provided as a pharmaceutical composition together with a pharmaceutically acceptable carrier. The pharmaceutical composition is administered in an effective amount to a subject having a disease associated with CD153, for example multiple sclerosis, or to a subject suspected of having such disease, similarly to the anti-CD153 antibody of the present disclosure. The pharmaceutical composition is administered, for example, by intravenous (IV) administration (e.g., injection or infusion).

### [Reference Example]

### Effect of mouse anti-mouse CD153 monoclonal antibody 17D on a multiple sclerosis model

The mouse anti-mouse CD153 monoclonal antibody 17D is an antibody that specifically binds to mouse antigen, and its epitope is presumed to exist at amino acid positions 154-177 and 197-217 of the extracellular region of mouse CD153. Using mice in which experimental autoimmune encephalomyelitis (EAE), an animal experimental model of multiple sclerosis, was induced, it was confirmed that the mouse anti-mouse CD153 monoclonal antibody 17D is effective in suppressing onset and exacerbation of multiple sclerosis.

The heavy chain and light chain sequences of the mouse anti-mouse CD153 monoclonal antibody 17D are as follows:
Heavy chain: SEQ ID NO: 7 
MSSPQTLNTLTLTMGWSWIFLFLLSGTAGVLSEVQLQQSGPELVKPGASVKISCKASGYTFTDYYMNWVKQSHGKTLEWIG
DINPNHGGATYNQKFKGKATLTVDKSSSTAYMELRSLTSEDSAVYYCASPYDGYYGFAYWGQGTLVTVSA
Light chain: SEQ ID NO: 8

MVSTPQFLVFLLFWIPASRGDILLTQSPAILSVSPGERVSFSCRASQSIGTSIHWYQQRTNGSPRLLIKYGSESISGIPS
RFSGSGSGTDFTLSINSVESEDIADYYCQQSNSWPTTFGGGTKLEIK

In each sequence, the boxed region indicates the signal sequence, and the underlined regions indicate the respective CDRs.

The sequences of the heavy chain and light chain CDRs of mouse anti-mouse CD153 monoclonal antibody 17D (hereinafter 17D) estimated based on the Kabat system are as follows:

**[Table 2]**

| | | SEQ ID NO | | | SEQ ID NO |
|---|---|---|---|---|---|
| CDR-H1 | DYYMN | 9 | CDR-L1 | RASQSIGTSIH | 12 |
| CDR-H2 | DINPNHGGATYNQKFKG | 10 | CDR-L2 | YGSESIS | 13 |
| CDR-H3 | PYDGYYGFAY | 11 | CDR-L3 | QQSNSWPTT | 14 |

### Materials and Methods

Thirteen-week-old female C57BL/6J mice (n = 10) were immunized subcutaneously (s.c.) with 200 µg of MOG35-55 peptide (MEVGWYRSPFSRVHLYRNGK) emulsified in complete Freund's adjuvant (CFA) containing 500 µg of Mycobacterium tuberculosis H37Ra. In addition, 500 ng of pertussis toxin was administered intraperitoneally on the day of immunization and two days thereafter. Either 100 µl (500 µg) of 17D antibody (n = 5) or 100 µl of PBS (n = 5) was administered intraperitoneally three times per week starting from the day before immunization.

### Clinical symptoms of EAE were evaluated daily according to the following EAE score:

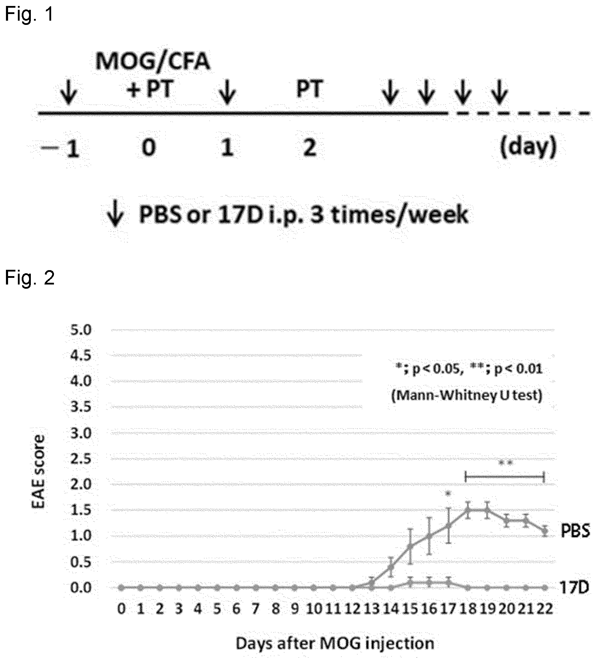

0, normal; 0.5, slight tail drooping; 1.0, complete tail drooping; 1.5, complete tail drooping and mild hind limb weakness; 2.0, complete tail drooping and moderate hind limb weakness; 2.5, complete tail drooping and severe hind limb weakness; 3.0, complete tail drooping and complete paralysis of both hind limbs; 3.5, complete tail drooping, complete paralysis of both hind limbs, and inability to right the body when the mouse is placed on its side, or both hind limbs together on one side of the body; 4.0, complete tail drooping, complete paralysis of both hind limbs, and partial paralysis of forelimbs; 4.5, complete tail drooping, complete paralysis of both hind limbs, partial paralysis of forelimbs, and almost no movement; 5, death or euthanasia due to severe paralysis. The schedule is summarized in Fig. 1.

### Statistical Analysis

Statistical analysis was performed using GraphPad Prism 9. For comparison between two groups, a nonparametric two-tailed Mann-Whitney U test was used. P < 0.05 was regarded as statistically significant. *; P < 0.05 and **; P < 0.01.

### Results

The results are shown in Fig. 2. All five mice administered PBS developed EAE around day 13 after MOG/CFA immunization, and the average score was highest (1.5) on days 18-19. In contrast, four mice administered 17D showed no signs of EAE throughout the experimental period. One mouse administered 17D developed very mild EAE (score 0.5), and recovered within three days.

### EAE incidence:

PBS: 5/5 (maximum score 2.0)
17D: 1/5 (maximum score 0.5)

The mouse anti-mouse CD153 antibody 17D prevented onset or exacerbation of EAE.

The present inventors also examined localization of SA-T cells in central nervous system tissue sections of EAE-induced mice obtained in the same manner as described above. As a result, PD-1-positive helper T cells including SA-T cells, which were not observed in healthy control mice, were confirmed to cluster near the meninges of the central nervous system or to infiltrate into the parenchyma (data not shown).

Taken together with the results of the above Reference Example and the finding shown in Fukushima et al., 2022, Cell Reports 40, 111373 that mouse anti-mouse CD153 monoclonal antibody 17D suppresses increase of SA-T cells, it is understood that increase of SA-T cells is related to multiple sclerosis, that increase of SA-T cells can be suppressed by anti-CD153 antibody, and that as a result anti-CD153 antibody is effective for treatment of multiple sclerosis.

### [Example 1]

### Acquisition of an anti-human CD153 monoclonal antibodies

### (1) Preparation of recombinant human (rh) CD153 protein

An expression vector for rhCD153 protein serving as antigen was constructed by inserting into p3xFLAG-CMV-9 a gene sequence in which a trimerization motif (SP-D) and an 8xHis-tag for purification were added to the extracellular region of hCD153. The constructed vector was transfected into FreeStyle^{™} 293-F cells and cultured in suspension at 37°C in an 8% CO₂ incubator for 3-4 days. The expressed protein in the culture supernatant was recovered using an affinity purification resin having affinity for the His-tag and further purified by gel filtration chromatography.

### (2) Immunization

Purified rhCD153 (100 µg) was mixed with complete Freund's adjuvant to form an emulsion and administered subcutaneously to female 8-week-old CD153-KO B6 mice (female). The rhCD153 (without adjuvant) was administered intraperitoneally as a boost at 3, 6, and 8 weeks after the first administration, and the mice were dissected three days after the final boost.

### (3) Preparation and screening of hybridomas

Splenocytes obtained from the spleen of the animals were fused with myeloma cells SP2/0 using PEG1500 and subjected to HAT selection culture in 96-well plates. Culture supernatants were collected from wells forming colonies and examined for production of anti-human CD153 antibody by ELISA (primary screening). Cells from wells showing positive reactions were cloned by limiting dilution to obtain hybridomas. Then, hybridoma culture supernatant was added to 293H cells expressing hCD153 and allowed to react for 30 minutes at room temperature. After washing, the cells were reacted with Alexa 488-labeled anti-mouse IgG for 30 minutes at room temperature, and clones positive for Alexa 488 fluorescence were selected by flow cytometry analysis (secondary screening). Antibodies were purified from hybridoma culture supernatants of three selected clones (2H7-9, 3A2-5, 13H9-13) using Protein A. Each purified antibody is hereinafter identified by the hybridoma clone name (2H7-9, 3A2-5, 13H9-13).

### (4) Evaluation of reactivity of purified antibodies

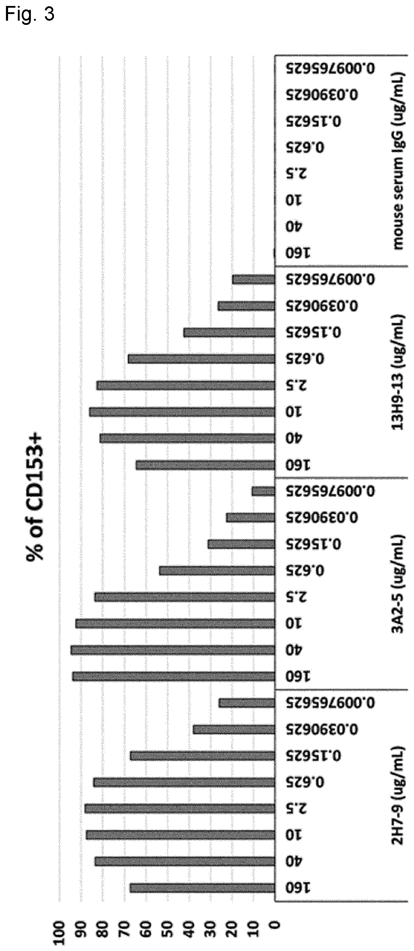

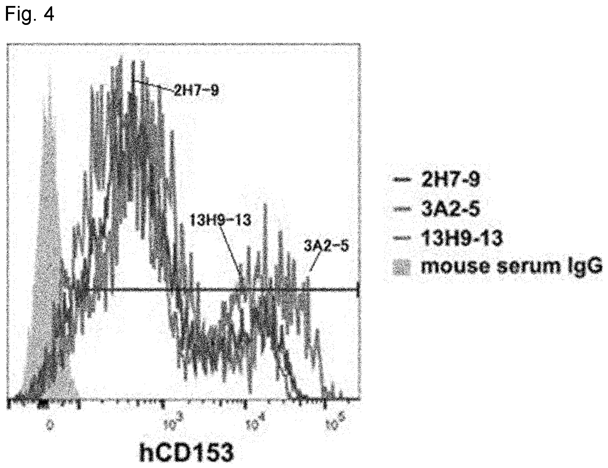

Reactivity of the purified antibodies was analyzed by flow cytometry. Purified antibody solutions serially diluted four-fold from 160 µg/ml to 0.00977 µg/ml and mouse serum IgG (negative control) were added to 293T cells transiently expressing human CD153 and allowed to react at 4°C for 20 minutes. After washing, cells were reacted with R-Phycoerythrin-labeled anti-mouse IgG at 4°C for 20 minutes, and fluorescence intensity was compared among clones. Results are shown in Figs. 3 and 4.

### (5) Reactivity to mouse CD153

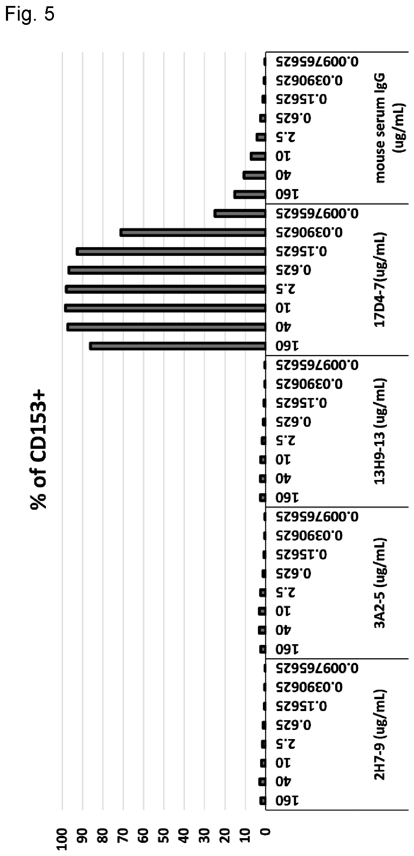

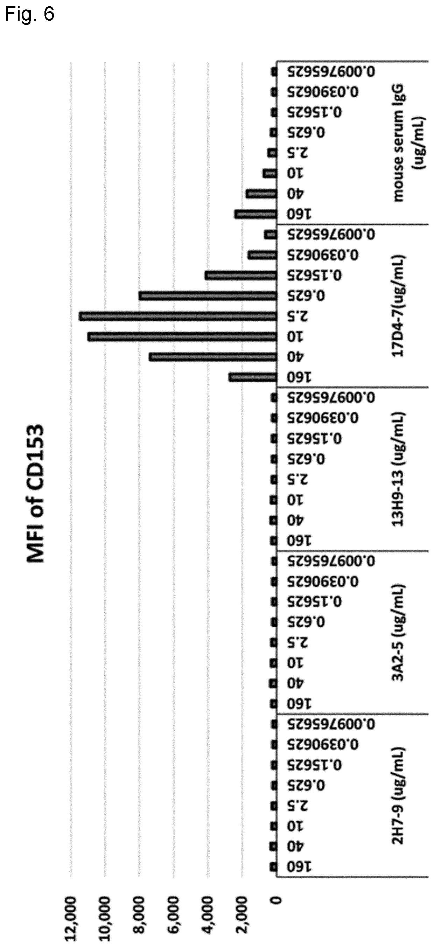

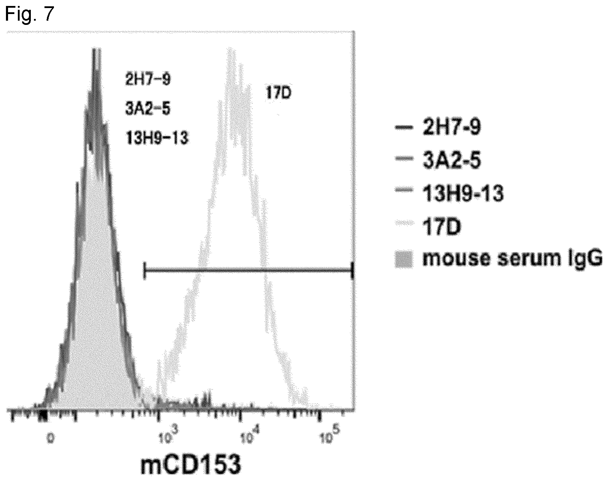

Except that EL4 cells stably expressing mouse CD153 were used, cross-reactivity to mouse CD153 was examined in the same manner as above. Mouse anti-mouse CD153 monoclonal antibody 17D was used as positive control. Results are shown in Figs. 5-7. Purified antibodies 2H7-9, 3A2-5, and 13H9-13 did not react with mouse CD153.

### (6) Evaluation of inhibitory effect on human CD153/CD30 interaction

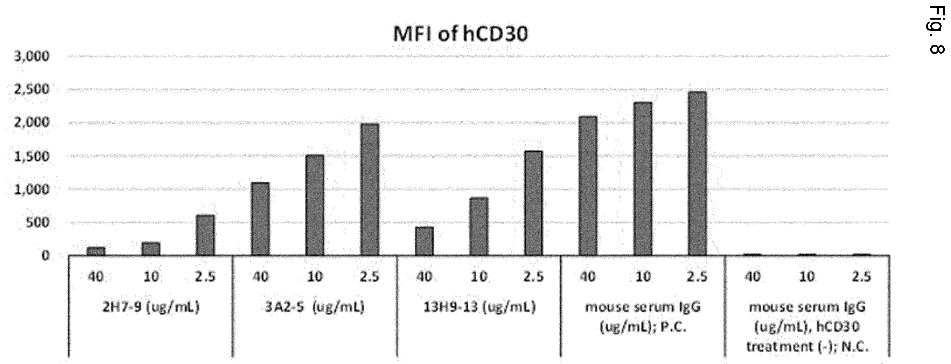

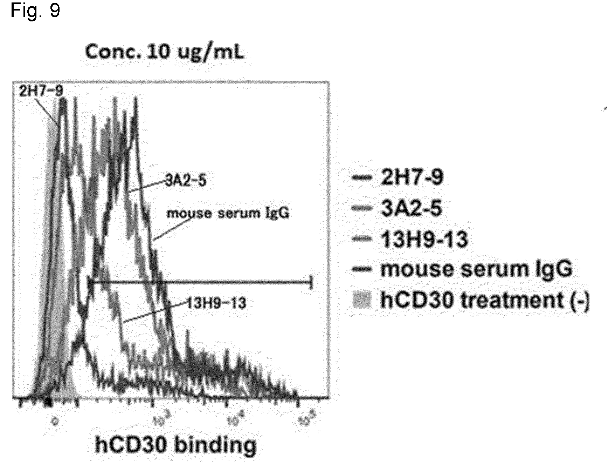

Purified anti-human CD153 antibody solutions diluted to 40, 10, and 2.5 µg/ml and mouse serum IgG were added to 293T cells transiently expressing human CD153 and allowed to react at 4°C for 20 minutes. After washing, 10 µg/ml rhCD30/human IgG1 chimeric protein (R&D, 813-CD-100) was added and reacted at 4°C for 30 minutes. After further washing, R-Phycoerythrin-labeled anti-human IgG was added and reacted at 4°C for 20 minutes. Decrease in fluorescence intensity was used as an indicator of inhibitory effect and compared among antibodies. Results are shown in Figs. 8 and 9.

Based on results of Example 1, among the three purified antibodies obtained, 2H7-9 was selected for further study. The heavy chain and light chain variable regions of monoclonal antibody 2H7-9 were determined, and respective CDRs were estimated by the Kabat method.
Heavy chain variable region: SEQ ID NO: 15 
MEWSGVFIFLLSVSTGVHSRVQLQQSGAELVRPGTSVKISCKASGYAFTNYWLGWVKQRPGHGLEWIGDIYPGNYNSYSN
EKFKGKATLTADESSSTAYMQLSSLTSEDSAVYFCARIYGNYWIFDVWGAGTTVTVSS
Light chain variable region: SEQ ID NO: 16

MHFQVQIFSFLLISASVIMSRGQIVLTQSPAIMSASPGEKVTMTCSASSSVNYMHWFQQKPGTSPKLWIYSTSDLASGVP
ARFSGSGSGTSYSLTISRMEAEDAATYYCQQRSSYPLTFGAGTKLELK

In each sequence, the boxed region indicates the signal sequence, and the underlined regions indicate the respective CDRs. Estimated CDRs are summarized in Table 3.

**[Table 3]**

| | | SEQ ID NO | | | SEQ ID NO |
|---|---|---|---|---|---|
| CDR-H1 | NYWLG | 1 | CDR-L1 | SASSSVNYMH | 4 |
| CDR-H2 | DIYPGNYNSYSNEKFKG | 2 | CDR-L2 | STSDLAS | 5 |
| CDR-H3 | IYGNYWIFDV | 3 | CDR-L3 | QQRSSYPLT | 6 |

### [Example 2]

### Evaluation of preventive and therapeutic effects of anti-human CD153 antibody (2H7-9) on EAE

Mice expressing hCD153 and hCD30 (hCD153/hCD30 knock-in mice) are generated by CRISPR/Cas-mediated genome editing inserting nucleotide sequences encoding hCD153 and hCD30 into endogenous mouse CD153 and CD30 loci, respectively. Female hCD153/hCD30 knock-in mice are immunized subcutaneously with myelin oligodendrocyte glycoprotein (MOG) peptide 35-55(MOG₃₃₋₅₅) emulsified in complete Freund's adjuvant containing Mycobacterium tuberculosis H37Ra, and pertussis toxin is injected intraperitoneally to induce EAE. 2H7-9 or PBS (or mouse serum IgG) is administered intraperitoneally before or after onset of EAE. Clinical symptoms are evaluated daily. Effect is evaluated by comparing incidence and severity between groups. 2H7-9 suppresses onset or exacerbation of EAE.

### [Example 3]

### Reactivity of anti-human CD153 antibodies (2H7-9, 3A2-5, 13H9-13) to canine and feline CD153

Expression vectors expressing canine CD153 and feline CD153 were constructed by inserting DNA sequences encoding canine CD153 protein and feline CD153 protein, respectively, into the pIRES2-AcGFP1 vector. The constructed vectors were transfected into 293T cells and cultured for two days at 37°C in a 5% CO₂ incubator. After culture, the cells were detached from the culture dish using trypsin-EDTA, washed with medium, and further washed with 2% FCS-PBS. Anti-human CD153 antibodies were added to the cells and allowed to react for 20 minutes at 4°C.

After washing the cells, R-Phycoerythrin-labeled anti-mouse IgG was added and allowed to react for 20 minutes at 4°C. Fluorescence of R-Phycoerythrin in vector-introduced cells (AcGFP1-positive fraction) was detected by FACS, and binding of each anti-human CD153 antibody to canine and feline CD153 was evaluated.

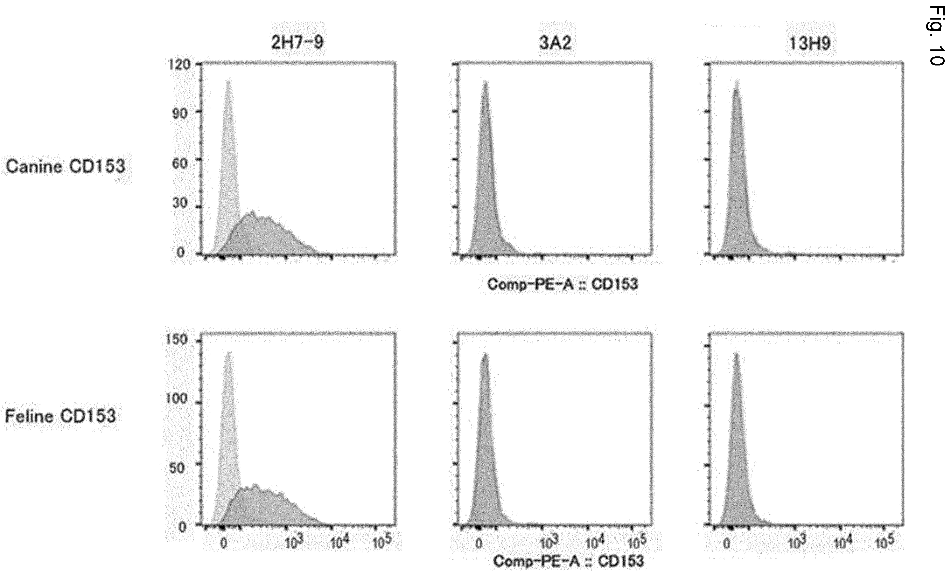

The results are shown in Fig. 10. It was confirmed that anti-human CD153 antibody 2H7-9 reacted with canine and feline CD153. In contrast, 3A2-5 and 13H9-13 did not react with either canine or feline CD153.

### [Example 4]

### Preparation of CD153 chimeric antigen receptor (CAR) T cells

As an expression vector for anti-mouse CD153 CAR protein, a retroviral vector was constructed by inserting a DNA sequence encoding a single-chain antibody sequence (scFv), in which the heavy chain variable region and light chain variable region of anti-mouse CD153 antibody clone 17D shown in the Reference Example were linked by a linker peptide, together with a hinge sequence, a transmembrane domain, a co-stimulatory molecule intracellular domain, and a CD3ζ intracellular domain.

The constructed retroviral vector was transfected into platinum-E cells. Culture supernatant containing viral particles was collected and used to infect CD8-positive T cells isolated from spleen cells of wild-type mice, thereby generating mCD153-CAR-T cells.

### [Example 5]

### Confirmation of cytotoxic effect of mCD153-CAR-T cells on CD153-expressing cells

The mCD153-CAR-T cells obtained in Example 4 were mixed with a T cell line stably expressing mCD153 (EGFP-positive) at an effector : target ratio of 10:1 or 3:1, and cultured at 37°C in a 5% CO₂ incubator. After 36 hours of culture, the cells were collected and stained with SYTOX Red dye to stain dead cells. The proportion of dead T cell line cells was measured by FACS. As a control, CD8-positive T cells isolated from spleen cells of wild-type mice without CAR introduction were used.

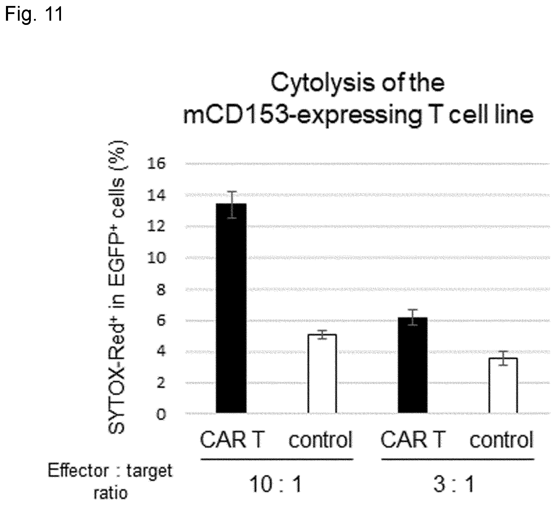

The results are shown in Fig. 11. Compared with the control group, mCD153-CAR-T cells markedly increased cell death of the mCD153-expressing T cell line, thereby confirming that they exert a cytotoxic effect against mCD153-expressing T cells. These results suggest that the increase of SA-T cells in multiple sclerosis can also be suppressed by administration of CAR-T cells.

### [Example 6]

An expression vector for anti-human CD153 CAR protein can be constructed using the scFv sequence derived from anti-human CD153 antibody clone 2H7-9 in the same manner as in Example 4. hCD153-CAR-T cells produced by introducing this vector into human CD8-positive T cells function on the same principle as mCD153-CAR-T cells and can kill hCD153-expressing T cells. Accordingly, hCD153-CAR-T cells exhibit cytotoxic effect against hCD153-expressing T cells by the same test as in Example 5, except that hCD153-expressing T cell lines are used.

## Claims

1. An anti-human CD153 monoclonal antibody, or a binding fragment thereof, which specifically binds to human CD153 and inhibits the intermolecular interaction between human CD153 and CD30.

2. The monoclonal antibody or binding fragment thereof according to claim 1, comprising the following heavy chain complementarity determining regions (CDR-H1, CDR-H2, CDR-H3) and light chain complementarity determining regions (CDR-L1, CDR-L2, CDR-L3).
| | | SEQ ID NO | | | SEQ ID NO |
|---|---|---|---|---|---|
| CDR-H1 | NYWLG | 1 | CDR-L1 | SASSSVNYMH | 4 |
| CDR-H2 | DIYPGNYNSYSNEKFKG | 2 | CDR-L2 | STSDLAS | 5 |
| CDR-H3 | IYGNYWIFDV | 3 | CDR-L3 | QQRSSYPLT | 6 |

3. The monoclonal antibody or binding fragment thereof according to claim 1 or 2, which further binds to feline CD153 or canine CD153.

4. A polynucleotide encoding the monoclonal antibody or antigen-binding fragment thereof according to any one of claims 1 to 3.

5. An expression vector comprising the polynucleotide according to claim 4.

6. A pharmaceutical composition for treating a disease associated with CD153, comprising the monoclonal antibody or binding fragment thereof according to any one of claims 1 to 3.

7. The pharmaceutical composition according to claim 6, wherein the disease associated with CD153 is multiple sclerosis.

8. A method for treating or preventing a disease associated with CD153 in a subject, comprising administering an effective amount of the monoclonal antibody or binding fragment thereof according to any one of claims 1 to 3 to a subject in need of the treatment.

9. The method according to claim 8, wherein the subject is a human.

10. The method according to claim 8, wherein the subject is a dog or a cat.

11. The method according to any one of claims 8 to 10, wherein the disease associated with CD153 is multiple sclerosis.

12. A chimeric antigen receptor comprising an extracellular antigen-binding domain, a transmembrane domain, and an intracellular signaling domain,
wherein the extracellular antigen-binding domain comprises a single-chain antibody comprising the following heavy chain complementarity determining regions (CDR-H1, CDR-H2, CDR-H3) and light chain complementarity determining regions (CDR-L1, CDR-L2, CDR-L3).
| | | SEQ ID NO | | | SEQ ID NO |
|---|---|---|---|---|---|
| CDR-H1 | NYWLG | 1 | CDR-L1 | SASSSVNYMH | 4 |
| CDR-H2 | DIYPGNYNSYSNEKFKG | 2 | CDR-L2 | STSDLAS | 5 |
| CDR-H3 | IYGNYWIFDV | 3 | CDR-L3 | QQRSSYPLT | 6 |

13. A polynucleotide encoding the chimeric antigen receptor according to claim 12.

14. A vector comprising the polynucleotide according to claim 13.

15. A CAR-T cell expressing the chimeric antigen receptor according to claim 12 and having antigen-specific cytotoxic activity.

16. A pharmaceutical composition for treating a disease associated with CD153, comprising the CAR-T cell according to claim 15.

17. The pharmaceutical composition according to claim 16, wherein the disease associated with CD153 is multiple sclerosis.

18. A method for treating or preventing a disease associated with CD153 in a subject, comprising administering an effective amount of the CAR-T cell according to claim 15 to a subject in need of the treatment.

19. The method according to claim 18, wherein the subject is a human.

20. The method according to claim 18, wherein the subject is a dog or a cat.

21. The method according to any one of claims 18 to 20, wherein the disease associated with CD153 is multiple sclerosis.
